(19) Europäisches Patentamt
European Patent Office
Office européen des brevets

(11) **EP 4 059 025 B1**

(12) **EUROPÄISCHE PATENTSCHRIFT**

(45) Veröffentlichungstag und Bekanntmachung des Hinweises auf die Patenterteilung:
**18.03.2026   Patentblatt 2026/12**

(21) Anmeldenummer: **20807693.5**

(22) Anmeldetag: **12.11.2020**

(51) Internationale Patentklassifikation (IPC):
***G16H 20/40*** *(2018.01)*

(52) Gemeinsame Patentklassifikation (CPC):
**G16H 20/40**

(86) Internationale Anmeldenummer:
**PCT/EP2020/081893**

(87) Internationale Veröffentlichungsnummer:
**WO 2021/094446 (20.05.2021 Gazette 2021/20)**

(54) **COMPUTERSYSTEM ZUM FESTLEGEN VON EINSTELLWERTEN EINER BLUTBEHANDLUNGSVORRICHTUNG**

COMPUTER SYSTEM FOR SPECIFYING SETTINGS OF A BLOOD TREATMENT DEVICE

SYSTÈME INFORMATIQUE POUR SPÉCIFIER DES RÉGLAGES D'UN DISPOSITIF DE TRAITEMENT DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.11.2019   DE 102019130433**

(43) Veröffentlichungstag der Anmeldung:
**21.09.2022   Patentblatt 2022/38**

(73) Patentinhaber: **Fresenius Medical Care Deutschland GmbH
61352 Bad Homburg (DE)**

(72) Erfinder: **KLEWINGHAUS, Juergen
61440 Oberursel (DE)**

(74) Vertreter: **Bobbert & Partner
Patentanwälte PartmbB
Postfach 1252
85422 Erding (DE)**

(56) Entgegenhaltungen:
**EP-A2- 2 762 179     EP-B1- 2 762 179**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Computersystem gemäß dem Oberbegriff des Anspruchs 1. Sie betrifft zudem eine Blutbehandlungsvorrichtung gemäß dem Oberbegriff des Anspruchs 8 und ein System gemäß dem Oberbegriff des Anspruchs 10. Sie betrifft ferner ein Verfahren gemäß dem Oberbegriff des Anspruchs 11, außerdem ein digitales Speichermedium gemäß Anspruch 13 sowie ein Computerprogramm-Produkt gemäß Anspruch 14. Zum Zwecke der Blutbehandlung sind an der hierfür verwendeten Blutbehandlungsvorrichtung Einstellwerte für variable Behandlungsparameter von einem Benutzer, z. B. dem Klinikpersonal, einzugeben. Die Veränderung solcher Einstellwerte kann der Benutzer mittels manueller Eingabe, z. B. an einem Bildschirm (Touchscreen) oder an anderen Schnittstellen, veranlassen.

Zum Erreichen vorbestimmter Behandlungsziele oder -vorgaben wie der renalen Dosis bei der Dialysebehandlung kann es erforderlich sein, dass der Benutzer eine Reihe von Einstellungswerten ermittelt oder verändert, die zum Teil in Wechselwirkung miteinander stehen, weshalb das Einstellen der Blutbehandlungsvorrichtung eine anspruchsvolle und auch fehlerbehaftete Tätigkeit sein kann.

Die Patentanmeldung EP 2 762 179 A2 als nächstliegender Stand der Technik offenbart eine Vorrichtung zur extrakorporalen Behandlung von Blut mit einer Steuereinheit, die so konfiguriert ist, dass sie Werte für zwei oder mehr Flüssigkeitsdurchflussraten auf der Grundlage einer vom Bediener eingestellten Flüssigkeitsdurchflussrate und eines verordneten Dosierungswertes berechnet.

Eine Aufgabe der vorliegenden Erfindung kann darin bestehen, ein Computersystem zum Unterstützen des Benutzers beim Festlegen von Einstellwerten einer Blutbehandlungsvorrichtung vorzuschlagen, außerdem eine Blutbehandlungsvorrichtung, die ein erfindungsgemäßes Computersystem aufweist, ein System und ein Verfahren. Ferner sollen ein digitales Speichermedium, ein Computerprogramm-Produkt sowie ein Computerprogramm vorgeschlagen werden.

Die erfindungsgemäße Aufgabe kann durch ein Computersystem mit den Merkmalen des Anspruchs 1 gelöst werden. Sie kann ferner durch eine Blutbehandlungsvorrichtung mit den Merkmalen des Anspruchs 8 sowie durch ein System mit den Merkmalen des Anspruchs 10 gelöst werden, außerdem durch ein Verfahren mit den Merkmalen des Anspruchs 11. Sie kann ferner gelöst werden durch ein digitales Speichermedium mit den Merkmalen des Anspruchs 13 sowie durch ein Computerprogramm-Produkt mit den Merkmalen des Anspruchs 14.

Das erfindungsgemäße Computersystem dient einem Benutzer (Arzt, Personal, usw.) zum Festlegen von Einstellwerten zu ihrer späteren Verwendung an einer Blutbehandlungsvorrichtung und somit dessen Unterstützung. Es weist zumindest eine erste Eingabeschnittstelle, eine zweite Eingabeschnittstelle sowie eine Ausgabeschnittstelle auf. Ferner weist es eine Rechenvorrichtung und eine Anzeigevorrichtung auf oder ist mit den vorstehenden Schnittstellen in Signalverbindung verbunden.

Die Rechenvorrichtung ist programmiert, um mittels der ersten Eingabeschnittstelle einen Zielwert, der auch ein Zielbereich sein kann, hier exemplarisch die renale Dosis, als Parameterwert für eine, vor allem spätere oder künftige, Behandlung eines Patienten mittels einer Blutbehandlungsvorrichtung einzulesen.

Ferner ist die Rechenvorrichtung programmiert, um einem Benutzer mittels der Anzeigevorrichtung, vorzugsweise in einer Graphik, einen von der Rechenvorrichtung errechneten Auswahlbereich anzuzeigen.

Dabei wird der Auswahlbereich von der Rechenvorrichtung unter Berücksichtigung des eingelesenen Zielwerts der renalen Dosis ermittelt. Beispielsweise kann mittels des Auswahlbereichs eine Vielzahl von Kombinationen aus oder mit genau einem Einstellwert für den Fluss der Blutpumpe einerseits und genau einem Einstellwert für den Fluss der Dialysierflüssigkeitspumpe andererseits anzeigt werden. Diese Einstellbereiche, sollten sie an der Blutbehandlungsvorrichtung eingestellt werden, erlauben (ceteris paribus) das Erreichen des Zielwerts nach den der Rechenvorrichtung vorliegenden Informationen.

Mittels einer zweiten Eingabeschnittstelle kann ein Auswählen einer, vorzugsweise einer beliebigen, der angezeigten Kombinationen durch den Benutzer erfolgen.

Die Rechenvorrichtung ist dabei weiter programmiert, um den Einstellwert für die Blutpumpe und den Einstellwert für die Dialysierflüssigkeitspumpe der ausgewählten Kombination mittels der Ausgabeschnittstelle auszugeben.

Die erfindungsgemäße Blutbehandlungsvorrichtung ist als Dialysevorrichtung ausgestaltet, welche ein erfindungsgemäßes Computersystem aufweist.

Das erfindungsgemäße System weist eine oder mehrere Blutbehandlungsvorrichtungen und ein erfindungsgemäßes Computersystem auf oder besteht hieraus.

Dabei ist die Blutbehandlungsvorrichtung vorzugsweise als Dialysevorrichtung ausgestaltet.

Die eine oder mehreren Blutbehandlungsvorrichtung(en) und das Computersystem liegen getrennt voneinander vor. Der Begriff "getrennt voneinander" kann hierbei z. B. eine räumliche, körperliche Trennung und/oder eine Trennung derart umfassen, dass zwischen Blutbehandlungsvorrichtung und Computersystem keine Signalkommunikation besteht.

Das erfindungsgemäße Verfahren zum Festlegen von Einstellwerten einer Blutbehandlungsvorrichtung (oder zum Unterstützen z. B. des medizinischen Personals bei der Findung oder dem Festlegen von geeigneten Einstellwerten) umfasst die folgenden Schritte:

a. Bereitstellen eines erfindungsgemäßen Computersystems, einer erfindungsgemäßen Blutbehandlungsvorrichtung oder eines erfindungsgemäßen Systems;

b. Einlesen eines Zielwerts und/oder eines Zielbereichs für einen Parameterwert für eine sich vorzugsweise nach Abschluss des erfindungsgemäßen Verfahrens anschließende Behandlung eines Patienten, beispielsweise für die renale Dosis, mittels einer Blutbehandlungsvorrichtung oder Eingeben dieses Parameterwerts mittels einer ersten Eingabeschnittstelle; die erste Eingabeschnittstelle kann optional als Einleseschnittstelle verwendet werden;

c. Anzeigen eines von der Rechenvorrichtung errechneten oder ermittelten Auswahlbereichs von Kombinationen von Einstellwerten, wobei das Anzeigen mittels der Anzeigevorrichtung erfolgt, beispielsweise in einer Graphik, z. B. in einem Diagramm;

d. Auswählen einer der mittels des Auswahlbereichs angezeigten Kombinationen durch den Benutzer mittels einer zweiten Eingabeschnittstelle; und

e. Ausgeben des Einstellwerts für die Blutpumpe und des Einstellwerts für die Dialysierflüssigkeitspumpe der ausgewählten Kombination mittels der Ausgabeschnittstelle.

Alles oben oder nachstehend zum erfindungsgemäßen Computersystem oder seinen Merkmalen Ausgeführte trifft analog ohne Einschränkung auch auf das erfindungsgemäße Verfahren oder seine möglichen Ausgestaltungen zu und umgekehrt. Daher wird an dieser Stelle zur Vermeidung von Wiederholungen jeweils auf Ausführungen und Definitionen verwiesen, die zum selben Gegenstand oder Begriff an beliebiger anderer Stelle hierin gemacht sind.

Ein erfindungsgemäßes digitales, insbesondere nichtflüchtiges, Speichermedium, insbesondere in Form eines maschinenlesbaren Trägers, insbesondere in Form einer Diskette, CD, DVD, EPROM, FRAM (Ferroelectric RAM) oder SSD (Solid-State-Drive), insbesondere mit elektronisch oder optisch auslesbaren, Steuersignalen, kann konfiguriert oder programmiert sein, um derart mit einem programmierbaren Computersystem zusammenwirken, dass ein Computersystem zu einem erfindungsgemäßen Computersystem umprogrammiert wird, z. B. wenn sein Speicherinhalt auf dem programmierbaren Computersystem abläuft.

Ein erfindungsgemäßes Computerprogramm-Produkt weist einen volatilen, flüchtigen oder auf einem maschinenlesbaren Träger gespeicherten Programmcode oder eine Signalwelle auf, konfiguriert um derart mit einer programmierbaren Computerkonfiguration eines Computersystems zusammenzuwirken, dass das Computersystem zu einem erfindungsgemäßen Computersystem umprogrammiert wird. Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. ein Client/Server-system, ein Cloud Computing System, etc.) oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sowie jeder andere hierin genannte Speicher oder jedes andere hierin genannte Speichermedium sein.

Unter einem Computerprogrammprodukt kann erfindungsgemäß auch eine programmierte Applikation (kurz: App), beispielsweise insbesondere für ein Smartphone, ein Tablet oder ein sonstiges mobiles Handgerät, verstanden werden.

Ein erfindungsgemäßes Computerprogramm weist einen Programmcode auf zur Veranlassung, dass ein Computersystem zu einem erfindungsgemäßen Computersystem umprogrammiert wird, wenn das Computerprogramm auf einem entsprechenden Computersystem abläuft. Unter einem Computerprogramm kann erfindungsgemäß beispielsweise ein physikalisches, vertriebsfähiges Software-Produkt verstanden werden, welches ein Programm aufweist.

Bei allen vorausgegangenen bzw. folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können eines oder mehrere der vorstehend oder im Folgenden genannten Merkmale aufweisen.

Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin eine Eignung oder ein Verfahrensschritt genannt ist, umfasst die vorliegende Erfindung vorzugsweise auch eine entsprechende Programmierung oder Konfigurierung einer geeigneten, insbesondere erfindungsgemäßen, Vorrichtung oder eines Abschnitts hiervon.

Wann immer hierin von einer Ausführungsform die Rede ist, so handelt es sich um eine beispielhafte, erfindungsgemäße Ausführungsform.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Der Auswahlbereich kann weniger als die technisch möglichen und/oder in der Graphik insgesamt angezeigten Kombinationen umfassen.

Das Auswählen der mittels des Auswahlbereichs angezeigten Kombinationen kann auch ein Markieren sein, beispielsweise mittels einer Computermaus. Das Auswählen kann optional das Verschieben eines bereits markierten Wertes bzw. einer bereits markierten Kombination auf einen neuen Wert bzw. eine neue Kombination umfassen.

Die erste und/oder die zweite Eingabeschnittstelle können jeweils ein entsprechend ausgestalteter Touchscreen, ein Drehschalter, ein Schieber, eine Tastatur oder dergleichen sein.

Die erste Eingabeschnittstelle und die zweite Eingabeschnittstelle können identisch sein, also durch nur eine, gemeinsame Eingabeschnittstelle verwirklicht sein. Sie können alternativ hierzu verschieden und/oder unabhängig voneinander sein.

Das Berücksichtigen des Zielwerts oder -bereichs beim Ermitteln oder Errechnen der Einstellwerte des Auswahlbereichs kann z. B. basierend auf bekannten Algorithmen erfolgen, die wiederum weitere Parameterwerte, z. B. aus Hilfstabellen, usw. mit einbeziehen können. Diese Algorithmen und/oder weitere Parameterwerte (sowie zu letzten gehörende, weitere Einstellwerte) können beispielsweise in einer Speichervorrichtung des Computersystems hinterlegt sein.

Der Zielwert kann vom Benutzer eingegeben werden oder von der Rechenvorrichtung mittels ihrer ersten Schnittstelle, die z. B. eine Schnittstelle zu einer Speichervorrichtung sein kann, ausgelesen werden.

Der Zielwert kann sich auf den zu behandelnden, konkreten Patienten beziehen.

Eine renale Dosis kann als Quotient aus Effluentfluss oder Filtratfluss einerseits und Patientengewicht andererseits festgelegt werden.

Bei der Ermittlung des Effluentflusses können, je nach Art der Therapie, verschiedene Flüsse (z. B. Dialysatfluss, Substituatfluss, Calciumfluss, Citratfluss, Netto-Ultrafiltration) zu berücksichtigen sein.

Die renale Dosis kann beispielsweise die renale Zieldosis (*TRD*) oder die effektive renale Dosis (*RRD*) sein.

Die renale Zieldosis (*TRD*) kann z. B. mittels der folgenden Formel errechnet werden:

$$TRD = \frac{PostDF + DF + NFRF + \dfrac{PreDF}{1 + \dfrac{PreDF}{BF}}}{m}$$

Hierbei gilt:

*BF*      Blutflussrate
*RF*      Dialysierflüssigkeitsflussrate
*NFRF*      Netto-Flüssigkeitsentzugsflussrate
*m*      Patientengewicht
*PostDF*      Post-Dilutionsflussrate

**PreDF**     Prä-Dilutionsflussrate.

Die effektive renale Dosis (**RRD**) kann in Abhängigkeit von der Therapiedauer *t* mittels der Formel

$$RRD = \frac{\dfrac{PostDV}{t} + \dfrac{DV}{t} + \dfrac{NFRV}{t} + \dfrac{\dfrac{PreDV}{t}}{1 + \dfrac{\frac{PreDV}{t}}{\frac{BV}{t}}}}{m}$$

errechnet werden, wobei gilt:

**BV**       verarbeitetes Blutvolumen
**DV**       Dialysierflüssigkeitsvolumen
**NFRV**     Netto-Flüssigkeitsentzugsvolumen
**m**        Patientengewicht
**PostDV**   Post-Dilutionsvolumen
**PreDV**    Prä-Dilutionsvolumen

In einigen Ausführungsformen ist die Rechenvorrichtung des Computersystems dergestalt programmiert, dass sie in der Lage ist, weitere Parameterwerte, z. B. desselben oder eines weiteren Parameters, einzulesen und basierend auf diesen Parameterwerten den Auswahlbereich der möglichen Einstellwerte weiter zu beschränken, zu segmentieren, zu unterteilen oder zu klassifizieren. Das Einlesen dieser weiteren Parameterwerte kann wie das Einlesen des Zielwerts erfolgen.

In manchen Ausführungsformen berücksichtigen oder betreffen die weiteren Parameter oder Parameterwerte eine für oder während der Behandlung angestrebte Antikoagulation, insbesondere eine Citrat-Calcium-Antikoagulation, oder eine angestrebte Bicarbonatkonzentration, z. B. im Serum.

In einigen Ausführungsformen ist oder umfasst das Computersystem ein mobiles Gerät. Es kann ein handgehaltenes Gerät, Handy, Smartphone, Tablet oder dergleichen sein oder umfassen.

In manchen Ausführungsformen umfasst das Computersystem ein Computerprogramm oder eine Applikation (kurz: App), die auf ein solches mobiles Gerät oder anderes Gerät geladen werden kann bzw. auf einem solchen Gerät gespeichert ist, ausgeführt werden kann, wird oder abläuft.

In manchen Ausführungsformen ist die Rechenvorrichtung des Computersystems programmiert, um den Einstellwert für die Blutpumpe und den Einstellwert für die Dialysierflüssigkeitspumpe der ausgewählten Kombination mittels der Anzeigevorrichtung dem Benutzer zur Kenntnisnahme anzuzeigen und/oder mittels der Ausgabeschnittstelle auszugeben, z. B. an eine Steuer- oder Regelvorrichtung einer Blutbehandlungsvorrichtung weiterzugeben. Diese kann wiederum die weitergegebenen Einstellwerte einlesen und/oder speichern.

Die weitergegebenen Einstellwerte können für eine anstehende Behandlungssitzung mittels der Blutbehandlungsvorrichtung von dieser herangezogen werden.

In einigen Ausführungsformen können Ausgabeschnittstelle und Anzeigevorrichtung identisch sein. Ein Ausgeben mittels Ausgabeschnittstelle kann somit ein Anzeigen mittels der Anzeigevorrichtung sein, und umgekehrt.

In manchen Ausführungsformen ist das erfindungsgemäße Computersystem programmiert, um Werte, welche eingestellte Flüsse und/oder die renale Dosis angeben, etwa den Zielwert für die renale Dosis und/oder die tatsächlichen Flüsse oder die Einstellwerte für die hierin genannten Pumpen, z. B. die Blutpumpe, die Dialysierflüssigkeitspumpe, die Substituatpumpe, die Calciumpumpe, die Citratpumpe, die Heparinpumpe und/oder andere Pumpen, auszugeben, etwa mittels vorstehend genannter Ausgabeschnittstelle.

Das Computersystem kann programmiert sein, diese Werte beispielsweise an ein Logistiksystem und/oder ein Abrechnungssystem zu übergegeben. Das Logistiksystem und/oder ein Abrechnungssystem, welche außerhalb des Computersystems vorliegen können, wohl aber Teil des erfindungsgemäßen Systems sein können, können ihrerseits programmiert sein, um Lieferströme, Bestellungen, Verbrauch und dergleichen zu überwachen, aufzuzeichnen, zu speichern, auszugeben, in Rechnung zu stellen und/oder auf andere Weise zu verarbeiten. So kann das Logistiksystem konfiguriert sein, um mittels vom Computersystem übermittelter Werte eine Nachbestellung von bei einer Blutbehandlungssitzung verbrauchten Flüssigkeiten wie Citratlösung, Dialysierflüssigkeit usw. und Lieferung an die Klink, in welcher die Blutbehandlung erfolgt ist, zu veranlassen. Das Abrechnungssystem kann die vom Computersystem übermittelten Werte zur Abrechnung der Leistungen, die die Klink um Zusammenhang mit der Blutbehandlungssitzung erbracht hat, zu verwenden. Beispielsweise können der Zielwert der renalen Dosis oder der Wert, der den Verbrauch an Flüssigkeiten wie Heparin, Citratlösung usw. widerspiegelt, wie der Heparinfluss, zur Grundlage für eine Abrechnung gegenüber dem Patienten oder einem Kostenträger wie einer Versicherungsgesellschaft gemacht werden.

In manchen Ausführungsformen ist das erfindungsgemäße Computersystem eine Steuer- oder Regelvorrichtung einer Blutbehandlungsvorrichtung oder steht mit dieser in Signalkommunikation.

Erfindungsgemäß kann in einigen Ausführungsformen auch vorgesehen sein, das erfindungsgemäße Computersystem in einer Steuer- oder Regelvorrichtung einer, z. B. herkömmlichen, Blutbehandlungsvorrichtung zu implementieren, womit die Blutbehandlungsvorrichtung wiederum zu einer erfindungsgemäßen Blutbehandlungsvorrichtung konfiguriert würde. In diesen Ausführungsformen könnten dann bereits vorhandene Ein- und Ausgabeschnittstellen sowie die Rechenvorrichtung der Blutbehandlungsvorrichtung vorteilhaft zum Verwirklichen des erfindungsgemäßen Computersystems genutzt werden.

In manchen Ausführungsformen kann das Computersystem eine serverbasierte Lösung sein oder umfassen, bei der der Benutzer beispielsweise über eine Website ein auf dem Server ablaufendes Programm aufrufen kann, um die Schritte des erfindungsgemäßen Verfahrens ablaufen zu lassen oder zu initiieren. Das Computersystem kann somit optional einen Server umfassen. Es kann Benutzerschnittstellen und/oder Benutzergeräte wie Rechner, Handy oder Tablet umfassen, welche eingerichtet sind, um mit dem Server zu kommunizieren.

In einigen Ausführungsformen ist die Steuer- oder Regelvorrichtung programmiert, die Blutbehandlungsvorrichtung anhand des Einstellwerts für die Blutpumpe und des Einstellwerts für die Dialysierflüssigkeitspumpe gemäß der ausgewählten Kombination zu steuern oder zu regeln, insbesondere nach erfolgter Auswahl.

In manchen Ausführungsformen ist die erfindungsgemäße Blutbehandlungsvorrichtung ausgestaltet als Hämodialysevorrichtung, Hämofiltrationsvorrichtung oder Hämodiafiltrationsvorrichtung, insbesondere als eine Vorrichtung für die akute, für die chronische oder für die kontinuierliche Nierenersatztherapie (CRRT = continuous renal replacement therapy).

In einigen Ausführungsformen umfasst das erfindungsgemäße Verfahren als weiteren Schritt das Anzeigen oder Ausgeben des Einstellwerts für die Blutpumpe und des Einstellwerts für die Dialysierflüssigkeitspumpe der ausgewählten Kombination. Das Anzeigen oder Ausgeben kann optional mittels einer Anzeigevorrichtung zur Kenntnisnahme und/oder Bestätigung durch den Benutzer erfolgen.

Das Bestätigen kann ein einfaches Betätigen einer "OK"-Schaltfläche oder dergleichen sein.

Das Bestätigen umfasst in einigen Ausführungsformen kein Eintippen, Eingeben, Auswählen usw. von Einstellwerten aus einer Vielzahl von Optionen oder gar deren Berechnung im Kopf.

In manchen Ausführungsformen umfasst das erfindungsgemäße Verfahren als weiteren Schritt das, vorzugsweise automatische, Steuern oder Regeln der Blutbehandlungsvorrichtung anhand des Einstellwerts für die Blutpumpe und des Einstellwerts für die Dialysierflüssigkeitspumpe der ausgewählten Kombination.

Dies kann sich z. B. unmittelbar an das Auswählen oder ein Bestätigen der gemachten Auswahl durch den Benutzer anschließen.

In einigen Ausführungsformen umfasst das erfindungsgemäße Verfahren als weiteren Schritt das Einlesen des Einstellwerts für die Blutpumpe und des Einstellwerts für die Dialysierflüssigkeitspumpe der ausgewählten Kombination in einen Speicher (dieser kann z. B. flüchtig oder permanent sein), z. B. der Blutbehandlungsvorrichtung, für eine nachfolgende oder noch zu beginnende Behandlung oder Behandlungssitzung mittels der Blutbehandlungsvorrichtung.

Weitere Parameterwerte können der Säuren/Basenhaushalt des Patienten oder die Zusammensetzung seines Bluts sein oder betreffen.

Wenn hierin von "programmiert" oder "konfiguriert" die Rede ist, so ist auch offenbart, diese Begriffe gegeneinander auszutauschen.

Manche oder alle erfindungsgemäßen Ausführungsformen können einen, mehrere oder alle der oben und/oder im Folgenden genannten Vorteile aufweisen.

Ein Vorteil der vorliegenden Erfindung kann darin bestehen, dem Benutzer, also zumeist dem behandelnden Arzt, die Entscheidung zu ermöglichen oder zu erleichtern, welche Einstellwerte bei der anstehenden oder aktuellen Blutbehandlung für Pumpen der Blutbehandlungsvorrichtung ausgewählt werden dürfen, um bei der Behandlung mit ihnen zugleich einen vorgegebenen Zielwert erzielen zu können, welcher nicht zugleich auch ein Zieleinstellwert einer der Pumpen ist. Da durch die Erfindung angesichts des Zielwerts ungünstige Kombinationen der zahlreichen, möglichen Kombinationen von vornherein ausgeschlossen werden können, vermindert dies die Komplexität der Aufgabe der Entscheidungsfindung für den Arzt erheblich.

Ein Vorteil der vorliegenden Erfindung kann somit weiter darin bestehen, dass das Verändern von Einstellwerten durch die erfindungsgemäße Vereinfachung vorteilhafterweise insbesondere auch dem unerfahreneren Benutzer mehr Sicherheit beim Einstellen geben kann.

Die Fehlerwahrscheinlichkeit bei der Findung von Einstellwerten kann erfindungsgemäß deutlich verringert werden, beispielsweise weil Kopfrechenschritte oder das Risiko von Denkfehlern durch den Benutzer wegfallen können.

Somit kann mittelbar auch die Patientensicherheit vorteilhafterweise erhöht werden.

Die Zuständigkeit und Verantwortung für die Findung oder Änderung der Einstellwerte kann hingegen vorteilhafterweise beim Benutzer verbleiben. Der Benutzer erhält somit zwar wertvolle Unterstützung durch das erfindungsgemäße Computersystem, ohne aber durch dessen eigenmächtige Handlung überrascht werden zu können.

Bleiben Blutbehandlungsvorrichtung und Computersystem getrennte Vorrichtungen, so können bestehende Blutbehandlungsvorrichtungen mit dem erfindungsgemäßen Computersystem zum erfindungsgemäßen System kombiniert werden. Da Eingriffe in die Steuerung der Blutbehandlungsvorrichtung hierbei nicht erforderlich sind, bedarf es auch keiner erneuten Zulassung der Blutbehandlungsvorrichtung als medizinisches Gerät.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren der Zeichnung gilt:

**Fig. 1**  zeigt in stark vereinfachter Darstellung ein erfindungsgemäßes System mit einer Blutbehandlungsvorrichtung neben einem erfindungsgemäßen Computersystem;

**Fig. 2**  zeigt ein Ablaufdiagramm einer beispielhaften Ausführungsform des erfindungsgemäßen Verfahrens;

**Fig. 3a**  zeigt Teile einer Graphik (oder einer graphischen Anzeige), wie sie dem Benutzer bei einer beispielhaften Ausführungsform des erfindungsgemäßen Computersystems oder erfindungsgemäßen Verfahrens mittels der Anzeigevorrichtung angezeigt werden könnte;

**Fig. 3b**  zeigt die Graphik der Fig. 3a mit einem markierten Auswahlbereich für neue Einstellwerte; und

**Fig. 3c**  zeigt die Graphik der Fig. 3b während eines Einstellvorgangs.

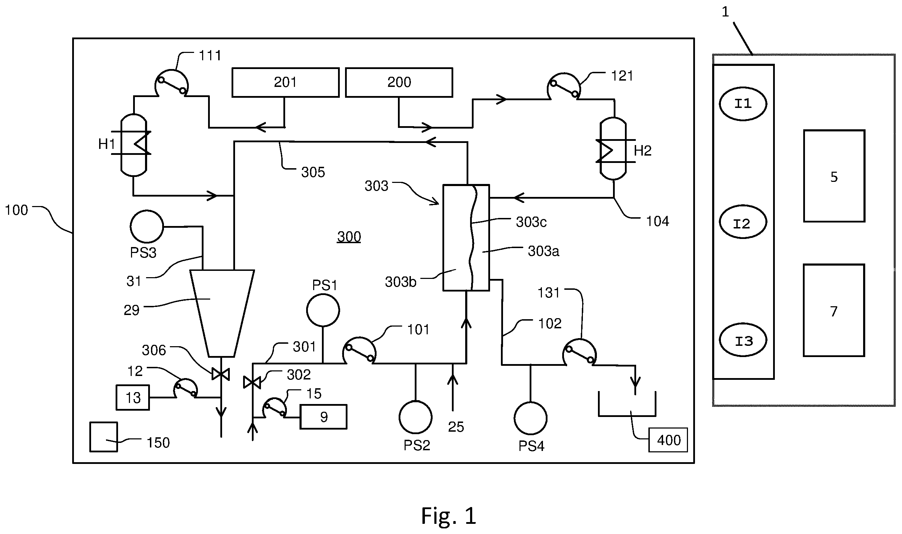

**Fig. 1** zeigt in stark vereinfachter Darstellung eine Blutbehandlungsvorrichtung 100, optional verbunden mit einem extrakorporalen Blutkreislauf 300, als Teil eines erfindungsgemäßen Systems.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter oder Dialysator 303. Der Blutfilter 303 weist eine Dialysierflüssigkeitskammer 303a und eine Blutkammer 303b auf, welche durch eine zumeist semi-permeable Membran 303c voneinander getrennt sind.

Der extrakorporale Blutkreislauf 300 weist ferner wenigstens eine zweite Leitung 305, hier in Form eines venösen Leitungsabschnitts, auf. Sowohl die erste Leitung 301 als auch die zweite Leitung 305 können zu ihrer Verbindung mit dem Gefäßsystem des nicht dargestellten Patienten dienen.

Die erste Leitung 301 ist optional mit einer (ersten) Schlauchklemme 302 zum Sperren oder Schließen der Leitung 301 verbunden. Die zweite Leitung 305 ist optional mit einer (zweiten) Schlauchklemme 306 zum Sperren oder Schließen der Leitung 305 verbunden.

Die in Fig. 1 nur durch einige ihrer Einrichtungen und schematisch repräsentierte Blutbehandlungsvorrichtung 100 weist eine Blutpumpe 101 auf. Die Blutpumpe 101 fördert während der Behandlung des Patienten Blut durch Abschnitte des extrakorporalen Blutkreislaufs 300 und in Richtung zum Blutfilter oder Dialysator 303, wie die kleinen Pfeilspitzen, welche in jeder der Figuren allgemein die Strömungsrichtung angeben, zeigen.

Mittels einer Pumpe für Dialysierflüssigkeit 121, die als Rollenpumpe oder als anderweitig okkludierende Pumpe ausgestaltet sein kann, wird frische Dialysierflüssigkeit aus einer Quelle 200 entlang der Dialysierflüssigkeitszulaufleitung 104 in die Dialysierflüssigkeitskammer 303a gepumpt. Die Dialysierflüssigkeit verlässt die Dialysierflüssigkeitskammer 303a als Dialysat, ggf. angereichert durch Filtrat, in Richtung eines optionalen Effluentbeutels 400 und wird hierin als Effluent bezeichnet.

Die Quelle 200 kann beispielsweise ein Beutel oder ein Container sein. Die Quelle 200 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 100.

Eine weitere Quelle 201 mit Substituat kann optional vorgesehen sein. Sie kann der Quelle 200 entsprechen oder eine eigene Quelle sein.

Eine nur angedeutete Steuer- oder Regelvorrichtung 150 kann dazu konfiguriert sein, die Blutbehandlungssitzung zu regeln oder zu steuern.

Rechts unten ist in Fig. 1 angedeutet, wo der optionale Effluentbeutel 400 mit der Blutbehandlungsvorrichtung 100 verbunden ist.

Neben der vorgenannten Blutpumpe 101 weist die in Fig. 1 gezeigte Anordnung ferner rein optional eine Reihe weiterer, jeweils optionaler Pumpen, nämlich die Pumpe 111 für Substituat, die Pumpe 121 für Dialysierflüssigkeit und die Pumpe 131 für das Effluent auf.

Die Pumpe 121 ist vorgesehen, um Dialysierflüssigkeit aus einer Quelle 200, beispielsweise einem Beutel, heraus und über eine optional vorhandene Beutelheizung H2 mit einem Heizbeutel dem Blutfilter 303 mittels der Dialysierflüssigkeitszulaufleitung 104 zuzuführen.

Die auf diese Weise zugeführte Dialysierflüssigkeit tritt über eine Dialysatablaufleitung 102, unterstützt durch die optionale Pumpe 131, wieder aus dem Blutfilter 303 aus und kann verworfen werden.

Stromauf der Blutpumpe 101 ist ein optionaler arterieller Sensor PS1 vorgesehen. Während einer Behandlung des Patienten misst er den Druck in der arteriellen Leitung.

Stromab der Blutpumpe 101, jedoch stromauf des Blutfilters 303 und, falls vorgesehen, stromaufwärts einer Zugabestelle 25 für Heparin, ist ein weiterer, optionaler Drucksensor PS2 vorgesehen. Er misst den Druck stromauf des Blutfilters 303 ("prä-Hämofilter").

Ein wiederum weiterer Drucksensor kann als PS4 stromab des Blutfilters 303, jedoch vorzugsweise stromauf der Pumpe 131, in der Dialysatablaufleitung 102 zum Messen des Filtratdrucks des Blutfilters 303 vorgesehen sein.

Blut, das den Blutfilter 303 verlässt, durchströmt eine optionale venöse Blutkammer 29, welche eine Entlüftungseinrichtung 31 und/oder einen weiteren Drucksensor PS3 aufweisen kann.

Die in Fig. 1 gezeigte Steuer- oder Regelvorrichtung 150 kann mit jeder der hierin genannten Komponenten - jedenfalls oder insbesondere mit der Blutpumpe 101 - in kabelgebundener oder kabelloser Signalverbindung zur Steuerung oder Regelung der Blutbehandlungsvorrichtung 100 stehen.

Die optionale Pumpe 111 ist vorgesehen, um Substituat aus der optionalen Quelle 201, beispielsweise einem Beutel, heraus und über eine optional vorhandene Beutelheizung H1 mit einem Heizbeutel der zweiten Leitung 305 zuzuführen.

Aus einer optional vorgesehenen Quelle für Citratlösung, hier beispielhaft als ein Citratbeutel 9 ausgestaltet, wird in manchen Ausführungsformen eine Citratlösung in die Leitung 301 abgegeben, gegebenenfalls mittels einer Citratpumpe 15. Aus der Quelle für Citratlösung wird beispielsweise 4%-iges $Na_3Citrat$ zugeführt.

Eine optionale Zugabeeinrichtung, hier als Calciumpumpe 12 ausgestaltet, ist vorgesehen, um aus einer optionalen Quelle für Calciumlösung, in Fig. 1 beispielhaft als ein Calciumbeutel 13 ausgestaltet, eine Calciumlösung in die Leitung 305 abzugeben. Aus der Quelle für Calciumlösung wird beispielsweise eine $CaCl_2$-Lösung zugeführt. Diese kann eine Calciumkonzentration von 91 mmol/l, 100 mmol/l oder eine andere geeignete Calciumkonzentration aufweisen.

Rechts neben der Blutbehandlungsvorrichtung 100 ist in der Fig. 1 ein erfindungsgemäßes Computersystem 1 mit einer Rechenvorrichtung 5 und einer optionalen Speichervorrichtung 7 dargestellt. Das Computersystem weist eine erste Eingabeschnittstelle I1 und eine zweite Eingabeschnittstelle I2 sowie eine Ausgabeschnittstelle I3 auf.

Blutbehandlungsvorrichtung 100 und Computersystem 1 stellen gemeinsam eine Ausführungsform eines erfindungsgemäßen Systems dar.

Das Computersystem 1 kann auf einem mobilen Handgerät, z. B. Handy oder Tablet, räumlich getrennt von der Blutbehandlungsvorrichtung 100 vorgesehen sein. Es ist in der in Fig. 1 gezeigten Ausführungsform nicht mit der Blutbehandlungsvorrichtung 100 in Signalkommunikation verbunden.

Das Computersystem 1 kann eine serverbasierte Lösung sein oder umfassen, bei der der Benutzer beispielsweise über eine Website ein auf dem Server ablaufendes Programm aufrufen kann, um die Schritte des erfindungsgemäßen Verfahrens ablaufen zu lassen oder zu initiieren. Das Computersystem 1 kann somit einen Server umfassen. Es kann Benutzerschnittstellen und/oder Benutzergeräte wie Rechner, Handy oder Tablet umfassen, welche eingerichtet sind, um mit dem Server zu kommunizieren.

In anderen Ausführungsformen der Blutbehandlungsvorrichtung als der hier gezeigten ist das Computersystem allerdings mit der Blutbehandlungsvorrichtung 100 in Signalkommunikation und/oder körperlich verbunden oder Teil hiervon. In letzteren Fällen handelt es sich bei den so verbundenen Vorrichtungen allerdings nicht mehr um ein erfindungsgemäßes System, sondern um eine erfindungsgemäße Blutbehandlungsvorrichtung.

Es ist weiterhin von der vorliegenden Erfindung umfasst, dass das Computersystem 1, anders als hier dargestellt, mit der Steuer- oder Regelvorrichtung 150 identisch ist oder von dieser umfasst wird.

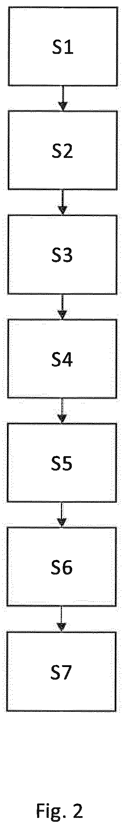

**Fig. 2** zeigt ein Ablaufdiagramm einer beispielhaften Ausführungsform des erfindungsgemäßen Verfahrens.

Dabei stellt der Verfahrensschritt S1 das Bereitstellen eines erfindungsgemäßen Computersystems 1, einer erfindungsgemäßen Blutbehandlungsvorrichtung 100 oder eines erfindungsgemäßen Systems dar.

Im Verfahrensschritt S2 wird ein Zielwert und/oder ein Zielbereich für einen Parameterwert für eine Behandlung eines Patienten, beispielsweise für die renale Dosis, mittels einer Blutbehandlungsvorrichtung 100 eingelesen oder dieser Parameterwert wird mittels einer ersten Eingabeschnittstelle I1 eingegeben.

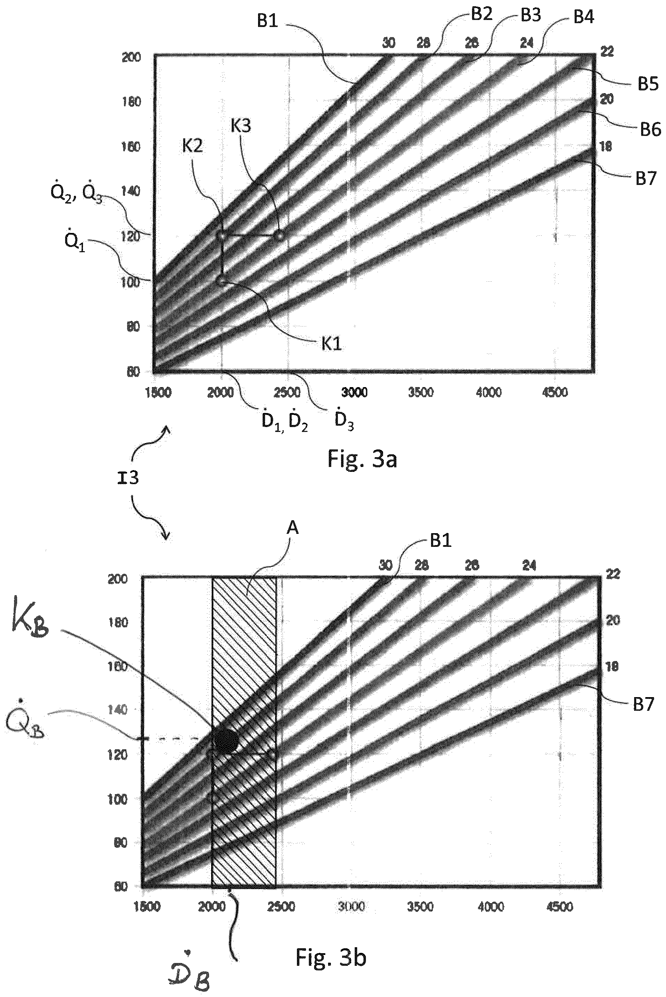

S3 repräsentiert das Anzeigen eines von der Rechenvorrichtung 5 errechneten Auswahlbereichs (siehe Bezugszeichen A in Fig. 3b), wie z. B. vorstehend beschrieben, mittels der Anzeigevorrichtung I3, beispielsweise in einer Graphik, z. B. einem Diagramm.

Im Verfahrensschritt S4 wird eine der angezeigten Kombinationen durch den Benutzer mittels einer zweiten Eingabeschnittstelle I2 ausgewählt, die wie in Fig. 3b gezeigt, optional identisch zur ersten Eingabeschnittstelle sein kann.

Im Verfahrensschritt S5 wird der Einstellwert für die Blutpumpe 101 und der Einstellwert für die Dialysierflüssigkeitspumpe 121 der ausgewählten Kombination mittels der Ausgabeschnittstelle ausgegeben.

Optional wird im Verfahrensschritt S6 der Einstellwert für die Blutpumpe 101 und des Einstellwerts für die Dialysierflüssigkeitspumpe 121 der ausgewählten Kombination mittels der Anzeigevorrichtung I3 angezeigt oder ausgegeben, damit sie der Benutzer zur Kenntnis nehmen kann.

S7 repräsentiert den optionalen Verfahrensschritt des Steuerns oder Regelns der Blutbehandlungsvorrichtung 100 anhand des Einstellwerts für die Blutpumpe 101 und des Einstellwerts für die Dialysierflüssigkeitspumpe 121 der ausgewählten Kombination.

**Fig. 3a** zeigt wesentliche Teile einer Graphik oder graphischen Anzeige, wie sie dem Benutzer bei einer beispielhaften Ausführungsform des Computersystems oder in einer beispielhaften Ausführungsform des Verfahrens mittels der Anzeigevorrichtung I3 angezeigt werden könnte.

Im Diagramm der graphischen Anzeige repräsentiert die vertikale Achse den Blutfluss [in ml/min] über dem Dialysierflüssigkeitsfluss [in ml/h] auf der horizontalen Achse.

Jeder Punkt innerhalb des gezeigten Diagramms entspricht somit einer Kombination aus einem möglichen Einstellwert $\dot{Q}_n$ für die Förderrate der Blutpumpe einerseits und einem möglichen Einstellwert $\dot{D}_n$ für die Förderrate der Dialysierflüssigkeitspumpe andererseits. So entspricht beispielsweise die Kombination K1 einer Kombination aus 100 ml/min als Rate für die Blutpumpe (Einstellwert $\dot{Q}_1$ = 100 ml/min) mit 2000 ml/min als Rate für die Dialysierflüssigkeitspumpe (Einstellwert $\dot{D}_1$ = 2000 ml/min). Die Kombination K2 hingegen steht für 120 ml/min als Rate für die Blutpumpe (Einstellwert $\dot{Q}2$ = 120 ml/min) mit - wie bereits in der Kombination K1 - 2000 ml/min als Rate für die Dialysierflüssigkeitspumpe (Einstellwert $\dot{D}_2$ = 2000 ml/min).

Bei der möglichen Einstellung einer Reihe solcher Kombinationen K1, K2, K3, ... aus einer Förderrate für die Blutpumpe und einer Förderrate für die Dialysierflüssigkeitspumpe ergibt sich rechnerisch ein erwarteter Säure-Basen-Status (erwartete Bicarbonatkonzentration im Serum).

Die zu erwartenden Bicarbonatkonzentrationen im Serum sind für eine Reihe von Kombinationen aus Blutflussrate und Dialysierflüssigkeitsflussrate jeweils in Strahlenform im Diagramm dargestellt. Die gezeigten Strahlen sind als B1 bis B7 bezeichnet. Für sie ist jeweils am Rand des Diagramms angegeben, welcher Wert (in mmol/l) an Bicarbonat bei Einstellen einer auf ihnen jeweils gelegenen Kombination an Flüssen zu erwarten ist (die Werte der Graphik der Fig. 3a wurden in einer Simulation ermittelt).

Am hier vorliegenden Beispiel ließe sich beispielsweise ablesen, dass eine Erhöhung des Blutflusses um 20 %, von K1 zu K2, die erwartete Bicarbonatkonzentration im Serum um etwa 4 mmol/l nach oben (von 24 mmol/l auf 28 mmol/l, d. h. in Richtung metabolische Alkalose) ändert. Eine Erhöhung des Dialysatflusses um ebenfalls 20 % (nach K3) würde diesen Effekt kompensieren.

Die zugrundeliegenden konstanten Behandlungsparameter sind in diesem Beispiel: Citratdosis 4 mmol pro Liter Blut, Calciumdosis 1,7 mmol pro Liter Blut, Netto-Ultrafiltration 100 ml/h.

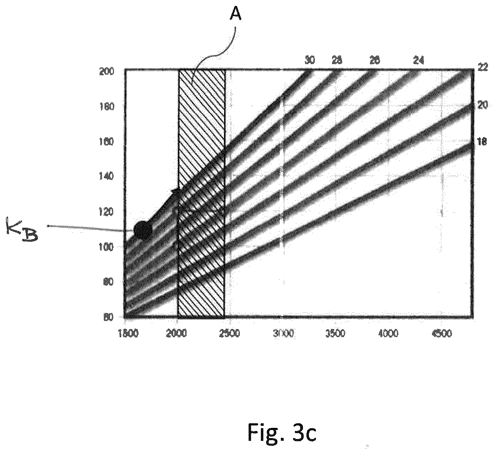

Der Auswahlbereich, der dem Benutzer erfindungsgemäß zur Verfügung steht, ist der Übersichtlichkeit halber in Fig. 3a noch nicht gezeigt. Er geht mit dem Bezugszeichen A aus Fig. 3b und Fig. 3c hervor.

**Fig. 3b** zeigt die beispielhafte Graphik oder graphische Anzeige der Fig. 3a.

Alle Ausführungen zu Fig. 3a treffen daher auch auf Fig. 3b zu.

In Fig. 3b ist hier ein Auswahlbereich A mit einer unbestimmten Anzahl n von möglichen Kombinationen von Einstellwerten $\dot{Q}_n$ für die Förderrate der Blutpumpe und Einstellwerten $\dot{D}_n$ für die Förderrate der Dialysierflüssigkeitspumpe dargestellt.

Der Auswahlbereich A stellt jene Kombinationen aus den n möglichen Kombinationen optisch heraus, bei welchen die zugehörigen Einstellwerte geeignet sind, an der Blutbehandlungsvorrichtung 100 eingestellt zu werden, um während oder bei der mit ihnen durchgeführten Behandlung den Zielwert, z. B. für die renale Dosis, erzielen zu können. Die mittels des Auswahlbereichs A markierten Kombinationen sind somit unter Berücksichtigung des Zielwerts für die renale Dosis ausgewählt oder ermittelt.

Der Auswahlbereich A ist im Beispiel der Fig. 3b durch Schraffur gekennzeichnet. Er muss nicht wie in der Fig. 3b gezeigt schraffiert dargestellt sein, sondern kann auch in anderer Art und Weise hervorgehoben werden. Farbliche Hinterlegungen sind ebenso möglich. Auch kann seine Form von der hier gezeigten Balkenform selbstverständlich abweichen.

Rein exemplarisch wird in Fig. 3b angenommen, der Benutzer habe sich z. B. mittels Markierens mit der Computermouse für eine Kombination $K_B$ entschieden, was anhand des Kreises innerhalb des Auswahlbereichs A dargestellt sein soll. Die dieser Kombination $K_B$ zugeordneten Einstellwerte $\dot{Q}_B$ für die Förderrate der Blutpumpe und $\dot{D}_B$ für die Förderrate der Dialysierflüssigkeitspumpe können dem Benutzer optional mit Angabe der genauen Einstellwerte in z. B. ml/min angezeigt werden. Es kann vorgesehen sein, dass der Benutzer sie zu bestätigen hat, bevor die Blutbehandlungsvorrichtung basierend hierauf tätig wird.

Ein Behandeln mittels der Einstellwerte $\dot{Q}_B$ $\dot{D}_B$ erlaubt jedoch ein Behandeln unter Erzielen der gewünschten renalen Dosis, also des Zielwertes.

Das Anzeigen oder Hervorheben der ausgewählten Kombination $K_B$ kann einem Ausgeben mittels der Ausgabeschnittstelle und/oder einem Anzeigen mittels der Anzeigevorrichtung entsprechen.

**Fig. 3c** zeigt das Diagramm der Fig. 3b. Abweichend von der Darstellung in Fig. 3b hat der Benutzer jedoch eine Kombination $K_B$ ausgewählt, die außerhalb des Auswahlbereichs A liegt. Die Einstellwerte dieser Kombination sind nicht geeignet, um bei einer mit ihnen durchgeführten Blutbehandlung den Zielwert für die renale Dosis zu erreichen.

Dem Benutzer kann dies optional mittels Warnung, Hinweises oder dergleichen zur Kenntnis gebracht werden. Er kann die Möglichkeit haben, seine Auswahl zu korrigieren und die zunächst ausgewählte Kombination $K_B$ in den Auswahlbereich A hinein zu verschieben. Folgt er dabei dem in Fig. 3c gezeigten Pfeil, so bliebe die von ihm angestrebte Bicarbonatkonzentration beibehalten.

**Bezugszeichenliste**

| | |
|---|---|
| 1 | Computersystem |
| 5 | Rechenvorrichtung |
| 7 | Speichervorrichtung (optional) |
| | |
| 9 | Quelle für Citratlösung, hier beispielhaft als ein Citratbeutel |
| 12 | Calciumpumpe |
| 13 | Quelle für Calciumlösung, Calciumbeutel |
| 15 | Citratpumpe |
| 25 | Zugabestelle für Heparin (optional) |
| 29 | venöse Blutkammer (optional) |
| 31 | Entlüftungseinrichtung |
| | |
| 100 | Blutbehandlungsvorrichtung |
| 101 | Blutpumpe |
| 102 | Dialysatablaufleitung, Effluentzulaufleitung |
| 104 | Dialysierflüssigkeitszulaufleitung |
| 111 | Pumpe für Substituat |
| 121 | Pumpe für Dialysierflüssigkeit |
| 131 | Pumpe für Dialysat oder Effluent in Effluentzulaufleitung |
| 150 | Steuer- oder Regelvorrichtung |
| | |
| 200 | Quelle mit Dialysierflüssigkeit |
| 201 | Quelle mit Substituat, optional |
| | |
| 300 | extrakorporaler Blutkreislauf |
| 301 | erste Leitung (arterieller Leitungsabschnitt) |
| 302 | (erste) Schlauchklemme |
| 303 | Blutfilter oder Dialysator |
| 303a | Dialysierflüssigkeitskammer |
| 303b | Blutkammer |
| 303c | semi-permeable Membran |
| 305 | zweite Leitung (venöser Leitungsabschnitt) |
| 306 | (zweite) Schlauchklemme |
| | |
| A | Auswahlbereich |
| I1 | erste Eingabeschnittstelle |
| I2 | zweite Eingabeschnittstelle |
| I3 | Anzeigevorrichtung |
| K1 | Kombination 1 |
| K2 | Kombination 2 |
| K3 | Kombination 3 |
| $K_B$ | ausgewählte Kombination |
| B1 | erwartete Bicarbonatkonzentration im Serum von 30 mmol/l |
| B2 | erwartete Bicarbonatkonzentration im Serum von 28 mmol/l |
| B3 | erwartete Bicarbonatkonzentration im Serum von 26 mmol/l |
| B4 | erwartete Bicarbonatkonzentration im Serum von 24 mmol/l |

| B5 | erwartete Bicarbonatkonzentration im Serum von 22 mmol/l |
| B6 | erwartete Bicarbonatkonzentration im Serum von 20 mmol/l |
| B7 | erwartete Bicarbonatkonzentration im Serum von 18 mmol/l |
| S1 | Verfahrensschritt a |
| S2 | Verfahrensschritt b |
| S3 | Verfahrensschritt c |
| S4 | Verfahrensschritt d |
| S5 | Verfahrensschritt e |
| S6 | weiterer Verfahrensschritt |
| S7 | weiterer Verfahrensschritt |
| $\dot{Q}_1, \dot{Q}_2, \dot{Q}_3, \dot{Q}_B$ | Einstellwerte für Blutpumpe |
| $\dot{D}_1, \dot{D}_2, \dot{D}_3, \dot{D}_B$ | Einstellwerte für Dialysierflüssigkeitspumpe |

**Patentansprüche**

1.  Computersystem (1) zum Festlegen von Einstellwerten einer Blutbehandlungsvorrichtung (100), mit

    - einer ersten Eingabeschnittstelle (I1);
    - einer zweiten Eingabeschnittstelle (I2);
    - einer Ausgabeschnittstelle;
    - einer Rechenvorrichtung (5);
    - einer Anzeigevorrichtung (I3)
    wobei die Rechenvorrichtung (5) programmiert ist,

    - um mittels der ersten Eingabeschnittstelle (I1) einen Zielwert für die renale Dosis als Parameterwert für eine Behandlung eines Patienten mittels einer Blutbehandlungsvorrichtung (100) einzulesen;
    - um einem Benutzer mittels der Anzeigevorrichtung (I3) in einer Graphik einen Auswahlbereich (A) anzuzeigen, wobei der Auswahlbereich (A) von der Rechenvorrichtung (5) unter Berücksichtigung des eingelesenen Zielwerts der renalen Dosis ermittelt wird und eine Vielzahl von Kombinationen ($K_1, K_2, K_3$) von je genau einem Einstellwert ($\dot{Q}_1, \dot{Q}_2, \dot{Q}_3$) für den Fluss der Blutpumpe (101) einerseits und genau einem Einstellwert ($\dot{D}_1, \dot{D}_2, \dot{D}_3$) für den Fluss der Dialysierflüssigkeitspumpe (121) andererseits anzeigt werden;

    wobei die zweite Eingabeschnittstelle (I2) ausgestaltet ist zum Auswählen einer Kombination ($\dot{K}_B$) aus den angezeigten Kombinationen (K1, K2, K3) durch den Benutzer; und
    wobei die Rechenvorrichtung (5) weiter programmiert ist,

    - um den Einstellwert ($\dot{Q}B$) für die Blutpumpe (101) und den Einstellwert (DB) für die Dialysierflüssigkeitspumpe (121) der ausgewählten Kombination (KB) mittels der Ausgabeschnittstelle auszugeben.

2.  Computersystem (1) nach Anspruch 1, wobei die Rechenvorrichtung (5) weiter programmiert ist,

    - um weitere Parameterwerte (B1, B2, B3, B4, B5, B6, B7) einzulesen;
    - wobei der Auswahlbereich (A) von der Rechenvorrichtung (5) unter Berücksichtigung der weiteren Parameterwerte (B1 bis B7) weiter beschränkt wird.

3.  Computersystem (1) nach Anspruch 2, wobei die weiteren Parameterwerte (B1 bis B7) eine angestrebte Antikoagulation, insbesondere Citrat-Calcium-Antikoagulation, oder eine angestrebte Bicarbonatkonzentration im Serum berücksichtigen oder betreffen.

4.  Computersystem (1) nach einem der vorangegangenen Ansprüche, wobei das Computersystem (1) ein mobiles Gerät, handgehaltenes Gerät, Handy, Smartphone, Tablet usw. oder eine hierfür geeignete Applikation ist.

5.  Computersystem (1) nach einem der vorangegangenen Ansprüche, wobei die Rechenvorrichtung (5) programmiert ist, den Einstellwert ($\dot{Q}_E$) für die Blutpumpe (101) und den Einstellwert ($\dot{D}_B$) für die Dialysierflüssigkeitspumpe (121) der ausgewählten Kombination ($K_B$) mittels der Anzeigevorrichtung (I3) dem Benutzer zur Kenntnisnahme anzuzeigen oder auszugeben.

6. Computersystem (1) nach einem der vorangegangenen Ansprüche, wobei das Computersystem (1) eine Steuer- oder Regelvorrichtung (150) einer Blutbehandlungsvorrichtung (100) ist oder mit dieser in Signalkommunikation steht.

7. Computersystem (1) nach Anspruch 6, wobei die Steuer-oder Regelvorrichtung (150) programmiert ist, die Blutbehandlungsvorrichtung (100) anhand des Einstellwerts ($\dot{Q}_B$) für die Blutpumpe (101) und des Einstellwerts ($\dot{D}_B$) für die Dialysierflüssigkeitspumpe (121) der ausgewählten Kombination ($K_B$) zu steuern oder zu regeln.

8. Blutbehandlungsvorrichtung (100), ausgestaltet als Dialysevorrichtung, aufweisend ein Computersystem (1) gemäß einem der vorangegangenen Ansprüche.

9. Blutbehandlungsvorrichtung (100) nach Anspruch 8, ausgestaltet als Hämodialysevorrichtung, Hämofiltrationsvorrichtung oder Hämodiafiltrationsvorrichtung, insbesondere als eine Vorrichtung für die chronische Nierenersatztherapie oder für die kontinuierliche Nierenersatztherapie (CRRT = continuous renal replacement therapy).

10. System aufweisend oder bestehend aus

   - einer oder mehreren Blutbehandlungsvorrichtungen (100), ausgestaltet als Dialysevorrichtung;
   - einem Computersystem (1) nach einem der vorangegangenen Ansprüche 1 bis 7,

   wobei die eine oder mehreren Blutbehandlungsvorrichtungen (100) und das Computersystem (1) getrennt voneinander vorliegen.

11. Verfahren zum Festlegen von Einstellwerten einer Blutbehandlungsvorrichtung (100) mit den Schritten:

   - Bereitstellen eines Computersystems (1) gemäß einem der Ansprüche 1 bis 7, einer Blutbehandlungsvorrichtung (100) gemäß einem der Ansprüche 8 bis 9, oder eines Systems gemäß Anspruch 10;
   - Einlesen eines Zielwerts für die renale Dosis als Parameterwert für eine Behandlung eines Patienten mittels einer Blutbehandlungsvorrichtung (100) oder Eingeben dieses Parameterwerts mittels der ersten Eingabeschnittstelle (I1);
   - Anzeigen, mittels der Anzeigevorrichtung (I3), in einer Graphik, eines Auswahlbereichs (A) zur Kenntnisnahme durch den Benutzer, wobei der Auswahlbereich (A) von der Rechenvorrichtung (5) unter Berücksichtigung des Zielwerts der renalen Dosis ermittelt wird und eine Vielzahl von Kombinationen je genau eines Einstellwerts ($\dot{Q}_1$, $\dot{Q}_2$, $\dot{Q}_3$) für den Fluss der Blutpumpe (101) einerseits und genau eines Einstellwerts ($\dot{D}_1$, $\dot{D}_2$, $\dot{D}_3$) für den Fluss der Dialysierflüssigkeitspumpe (121) andererseits anzeigt;
   - Auswählen einer Kombination ($K_B$) der angezeigten Kombinationen (K1, K2, K3) durch den Benutzer mittels der zweiten Eingabeschnittstelle (I2); und
   - Ausgeben des Einstellwerts ($\dot{Q}_B$) für die Blutpumpe (101) und des Einstellwerts ($\dot{D}_B$) für die Dialysierflüssigkeitspumpe (121) der ausgewählten Kombination ($K_B$) mittels der Ausgabeschnittstelle.

12. Verfahren nach Anspruch 11, mit dem weiteren Schritt:

   - Anzeigen oder Ausgeben des Einstellwerts ($\dot{Q}_B$) für die Blutpumpe (101) und des Einstellwerts ($\dot{D}_B$) für die Dialysierflüssigkeitspumpe (121) der ausgewählten Kombination ($\dot{K}_B$) mittels der Anzeigevorrichtung (I3) zur Kenntnisnahme durch den Benutzer.

13. Digitales Speichermedium, insbesondere in Form einer Diskette, CD oder DVD, EPROM, FRAM oder SSD, mit elektronisch auslesbaren Steuersignalen, konfiguriert, um derart mit einem programmierbaren Computersystem zusammenzuwirken, dass das Computersystem zu einem Computersystem gemäß der Ansprüche 1 bis 7 programmiert wird.

14. Computerprogramm-Produkt, als Signalwelle oder mit einem auf einem maschinenlesbaren Träger gespeicherten Programmcode, um derart mit einem programmierbaren Computersystem zusammenzuwirken, dass das Computersystem zu einem Computersystem gemäß der Ansprüche 1 bis 7 programmiert wird.

**Claims**

1. A computer system (1) for setting values of a blood treatment apparatus (100), comprising:

   - a first input interface (I1);
   - a second input interface (I2);
   - an output interface;
   - a calculation device (5);
   - a display device (I3)
   wherein the calculation device (5) is programmed:

   - to read in, by means of the first input interface (I1), a target value for the renal dose as a parameter value for a treatment of a patient by means of the blood treatment apparatus (100);
   - to display to a user a selection range (A) in a graphic, by means of the display device (I3), wherein the selection range (A) is determined by the calculation device (5) taking into account the read-in target value of the renal dose and a plurality of combinations $(K_1, K_2, K_3)$ of exactly one adjustment value $(\dot{Q}_1, \dot{Q}_2, \dot{Q}_3)$ for the flow of the blood pump (101) on the one hand and exactly one adjustment value $(\dot{D}_1, \dot{D}_2, \dot{D}_3)$ for the flow of the dialysis fluid pump (121) on the other hand are displayed;

   wherein the second input interface (I2) is designed to allow the user to select a combination $(K_B)$ from the displayed combinations $(K_1, K_2, K_3)$; and
   wherein the calculation device (5) is further programmed:

   - to output the adjustment value $(\dot{Q}_B)$ for the blood pump (101) and the adjustment value $(\dot{D}_B)$ for the dialysis fluid pump (121) of the selected combination $(K_B)$ by means of the output interface.

2. The computer system (1) according to claim 1, wherein the calculation device (5) is further programmed,

   - to read in further parameter values (B1, B2, B3, B4, B5, B6, B7);
   - wherein the selection range (A) is further restricted by the calculation device (5) taking into account the further parameter values (B1 to B7).

3. The computer system (1) according to claim 2, wherein the further parameter values (B1 to B7) take into account or relate to a desired anticoagulation, in particular citrate-calcium anticoagulation, or a desired bicarbonate concentration in the serum.

4. The computer system (1) according to any one of the preceding claims, wherein the computer system (1) is a mobile device, a handheld device, a mobile phone, a smartphone, a tablet, etc. or an application suitable for this purpose.

5. The computer system (1) according to any one of the preceding claims, wherein the calculation device (5) is programmed to display or output, for the user's attention, by means of the display device (I3), the adjustment value $(\dot{Q}_B)$ for the blood pump (101) and the adjustment value $(\dot{D}_B)$ for the dialysis fluid pump (121) of the selected combination $(K_B)$.

6. The computer system (1) according to any one of the preceding claims, wherein the computer system (1) is a control or regulating device (150) of the blood treatment apparatus (100) or is in signal communication therewith.

7. The computer system (1) according to claim 6, wherein the control or regulating device (150) is programmed to control or regulate the blood treatment apparatus (100) on the basis of the adjustment value $(\dot{Q}_B)$ for the blood pump (101) and the adjustment value $(\dot{D}_B)$ for the dialysis fluid pump (121) of the selected combination $(K_B)$.

8. A blood treatment apparatus (100), designed as a dialysis apparatus, comprising a computer system (1) according to any one of the preceding claims.

9. The blood treatment apparatus (100) according to claim 8, designed as a hemodialysis apparatus, hemofiltration apparatus or hemodiafiltration apparatus, in particular as an apparatus for chronic renal replacement therapy or for continuous renal replacement therapy (CRRT).

**10.** A system comprising or consisting of:

- one or more blood treatment apparatuses (100), designed as a dialysis apparatus;
- a computer system (1) according to any one of the preceding claims 1 to 7,

wherein the one or more blood treatment devices (100) and the computer system (1) are separate from one another.

**11.** A method for setting adjustment values of a blood treatment apparatus (100) comprising the steps of:

- providing a computer system (1) according to any one of claims 1 to 7, a blood treatment apparatus (100) according to any one of claims 8 to 9, or a system according to claim 10;
- reading in, by means of the first input interface (I1), a target value for the renal dose as a parameter value for a treatment of a patient by means of the blood treatment apparatus (100) or entering this parameter value;
- displaying to the user a selection range (A) in a graphic, by means of the display device (I3), wherein the selection range (A) is determined by the calculation device (5) taking into account the target value of the renal dose and displays a plurality of combinations of exactly one adjustment value ($\dot{Q}_1$, $\dot{Q}_2$, $\dot{Q}_3$) for the flow of the blood pump (101) on the one hand and exactly one adjustment value ($\dot{D}_1$, $\dot{D}_2$, $\dot{D}_3$) for the flow of the dialysis fluid pump (121) on the other hand;
- selecting by the user, by means of the second input interface (I2), a combination ($K_B$) from the displayed combinations ($K_1$, $K_2$, $K_3$); and
- outputting, by means of the output interface, the adjustment value ($\dot{Q}_B$) for the blood pump (101) and the adjustment value ($\dot{D}_B$) for the dialysis fluid pump (121) of the selected combination ($K_B$).

**12.** The method according to claim 11, with the further step of:

- displaying or outputting, for the user to acknowledge, by means the display device (I3), the adjustment value ($\dot{Q}_B$) for the blood pump (101) and the adjustment value ($\dot{D}_B$) for the dialysis fluid pump (121) of the selected combination ($K_B$).

**13.** A digital storage medium, in particular in the form of a floppy disk, CD or DVD, EPROM, FRAM or SSD, with electronically readable control signals, configured to interact with a programmable computer system in such a way that the computer system is programmed to become a computer system according to claims 1 to 7.

**14.** A computer program product, as a signal wave or with a program code stored on a machine-readable carrier, to interact with a programmable computer system in such a way that the computer system is programmed to become a computer system according to claims 1 to 7.

**Revendications**

**1.** Un système informatique (1) destiné à définir des valeurs de réglage d'un appareil de traitement du sang (100), comprenant :

- une première interface d'entrée (I1);
- une seconde interface d'entrée (I2);
- une interface de sortie;
- un dispositif de calcul (5);
- un dispositif d'affichage (I3)

où le dispositif de calcul (5) est programmé pour :

- lire, via la première interface d'entrée (I1), une valeur cible pour la dose rénale en tant que valeur de paramètre pour un traitement d'un patient au moyen de l'appareil de traitement du sang (100);
- afficher à un utilisateur une plage de sélection (A) dans un graphique, au moyen du dispositif d'affichage (I3), où la plage de sélection (A) est déterminée par le dispositif de calcul (5) en tenant compte de la valeur cible lue de la dose rénale et une pluralité de combinaisons ($K_1$, $K_2$, $K_3$) d'une seule valeur de réglage ($\dot{Q}_1$, $\dot{Q}_2$, $\dot{Q}_3$) pour le débit de la pompe à sang (101) d'une part et d'une seule valeur de réglage ($\dot{D}_1$, $\dot{D}_2$, $\dot{D}_3$) pour le débit de la pompe à liquide de dialyse (121) d'autre part sont affichées;

où la seconde interface d'entrée (I2) est conçue pour permettre à l'utilisateur de sélectionner une combinaison ($\dot{K}_B$) parmi les combinaisons affichées (K$_1$, K$_2$, K$_3$); et
où le dispositif de calcul (5) est en outre programmé pour :

- transmettre, au moyen de l'interface de sortie, la valeur de réglage ($\dot{Q}_B$) pour la pompe à sang (101) ainsi que la valeur de réglage ($\dot{D}_B$) pour la pompe à liquide de dialyse (121) de la combinaison sélectionnée (K$_B$).

2. Le système informatique (1) selon la première revendication, où le dispositif de calcul (5) est en outre programmé :

- pour lire des valeurs de paramètres supplémentaires (B1, B2, B3, B4, B5, B6, B7);
- où la plage de sélection (A) est en outre restreinte par le dispositif de calcul (5) en tenant compte des valeurs de paramètres supplémentaires (B1 à B7).

3. Le système informatique (1) selon la revendication 2, où les valeurs des paramètres supplémentaires (B1 à B7) prennent en compte ou se rapportent à une anticoagulation souhaitée, notamment une anticoagulation au citrate-calcium, ou une concentration de bicarbonate souhaitée dans le sérum.

4. Le système informatique (1) selon l'une quelconque des revendications précédentes, où le système informatique (1) est un appareil mobile, un appareil portable, un téléphone portable, un smartphone, une tablette, etc. ou une application appropriée à cette fin.

5. Le système informatique (1) selon l'une quelconque des revendications précédentes, où le dispositif de calcul (5) est programmé pour afficher ou transmettre, à l'attention de l'utilisateur, via le dispositif d'affichage (I3), la valeur de réglage ($\dot{Q}_B$) pour la pompe à sang (101) ainsi que la valeur de réglage ($\dot{D}_B$) pour la pompe à liquide de dialyse (121) de la combinaison sélectionnée (K$_B$).

6. Le système informatique (1) selon l'une quelconque des revendications précédentes, où le système informatique (1) est un dispositif de commande ou de régulation (150) de l'appareil de traitement du sang (100), ou est en communication de signal avec celui-ci.

7. Le système informatique (1) selon la revendication 6, où le dispositif de commande ou de régulation (150) est programmé pour commander ou réguler l'appareil de traitement du sang (100) en fonction de la valeur de réglage ($\dot{Q}_B$) de la pompe à sang (101) ainsi que de la valeur de réglage ($\dot{D}_B$) de la pompe à liquide de dialyse (121) de la combinaison sélectionnée (K$_B$).

8. Un appareil de traitement du sang (100), conçu comme un appareil de dialyse, comprenant un système informatique (1) selon l'une quelconque des revendications précédentes.

9. L'appareil de traitement du sang (100) selon la revendication 8, conçu comme un appareil d'hémodialyse, un appareil d'hémofiltration ou un appareil d'hémodiafiltration, notamment comme un appareil pour la thérapie de remplacement rénal chronique ou pour la thérapie de remplacement rénal continue (CRRT = continuous renal replacement therapy).

10. Un système comprenant ou constitué de :

- un ou plusieurs appareil(s) de traitement du sang (100) conçu(s) comme un appareil de dialyse;
- un système informatique (1) selon l'une quelconque des revendications 1 à 7 précédentes,

où le ou les appareil(s) de traitement du sang (100) et le système informatique (1) sont séparés les uns des autres.

11. Un procédé pour définir des valeurs de réglage d'un appareil de traitement du sang (100) comprenant les étapes suivantes :

- fournir un système informatique (1) selon l'une quelconque des revendications 1 à 7, un appareil de traitement du sang (100) selon l'une quelconque des revendications 8 à 9, ou un système selon la revendication 10;
- lire, via la première interface d'entrée (I1), une valeur cible pour la dose rénale en tant que valeur de paramètre pour un traitement d'un patient au moyen de l'appareil de traitement du sang (100) ou entrer cette valeur de paramètre;

- afficher, à la connaissance de l'utilisateur, une plage de sélection (A) dans un graphique, au moyen du dispositif d'affichage (I3), où la plage de sélection (A) est déterminée par le dispositif de calcul (5) en tenant compte de la valeur cible de la dose rénale et affiche une pluralité

de combinaisons d'une seule valeur de réglage ($\dot{Q}_1$, $\dot{Q}_2$, $\dot{Q}_3$) pour le débit de la pompe à sang (101) d'une part et d'une seule valeur de réglage ($\dot{D}_1$, $\dot{D}_2$, $\dot{D}_3$) pour le débit de la pompe à liquide de dialyse (121) d'autre part;
- sélectionner par l'utilisateur, au moyen de la seconde interface d'entrée (I2), une combinaison ($K_B$) parmi les combinaisons affichées ($K_1$, $K_2$, $K_3$); et
- transmettre, au moyen de l'interface de sortie, la valeur

de réglage ($\dot{Q}_B$) pour la pompe à sang (101) ainsi que la valeur de réglage ($\dot{D}_B$) pour la pompe à liquide de dialyse (121) de la combinaison sélectionnée ($K_B$).

**12.** Le procédé selon la revendication 11, comprenant l'étape supplémentaire suivante :

- afficher ou transmettre, à la connaissance de l'utilisateur, via le dispositif d'affichage (I3), la valeur de réglage ($\dot{Q}_B$) pour la pompe à sang (101) ainsi que la valeur de réglage ($\dot{D}_B$) pour la pompe à liquide de dialyse (121) de la combinaison sélectionnée ($K_B$).

**13.** Un support de stockage numérique, notamment sous la forme d'une disquette, d'un CD ou d'un DVD, d'une EPROM, d'une FRAM ou d'un SSD, avec des signaux de contrôle lisibles électroniquement, configuré pour interagir avec un système informatique programmable de manière à ce que le système informatique soit programmé pour devenir un système informatique selon les revendications 1 à 7.

**14.** Un produit de programme informatique, en tant qu'onde de signal ou avec un code de programme stocké sur un support lisible par machine, pour interagir avec un système informatique programmable de manière à ce que le système informatique soit programmé pour devenir un système informatique selon les revendications 1 à 7.

Fig. 1

EP 4 059 025 B1

Fig. 2

Fig. 3a

Fig. 3b

Fig. 3c

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2762179 A2